(19) Europäisches Patentamt
European Patent Office
Office européen des brevets

(11) Veröffentlichungsnummer: **0 477 639 B1**

# (12) EUROPÄISCHE PATENTSCHRIFT

(45) Veröffentlichungstag der Patentschrift: **12.04.95**

(51) Int. Cl.[6]: **C07C 271/54**, C07C 271/22, A01N 47/22

(21) Anmeldenummer: **91115186.8**

(22) Anmeldetag: **09.09.91**

## (54) Substituierte Aminosäureamid-Derivate deren Herstellung und Verwendung.

(30) Priorität: **22.09.90 DE 4030062**

(43) Veröffentlichungstag der Anmeldung:
**01.04.92 Patentblatt 92/14**

(45) Bekanntmachung des Hinweises auf die Patenterteilung:
**12.04.95 Patentblatt 95/15**

(84) Benannte Vertragsstaaten:
**BE CH DE ES FR GB GR IT LI NL**

(56) Entgegenhaltungen:
**EP-A- 0 398 072**
**EP-A- 0 425 925**
**GB-A- 873 049**

**CHEMICAL ABSTRACTS, Band 109, Nr. 11, 12.September 1988, Seite 773, Zusammenfassung Nr. 93603f, Columbus, Ohio, US; T. TSICHIYA et al, "Preparation of amino acid derivatives as sweetening agents"; & JP - A - 62252754**

(73) Patentinhaber: **BAYER AG**

**D-51368 Leverkusen (DE)**

(72) Erfinder: **Seitz, Thomas, Dr.**
**Mozartstrasse 32**
**W-4019 Monheim (DE)**
Erfinder: **Bender, Wolfgang, Dr.**
**Claudiusweg 5**
**W-5600 Wuppertal 1 (DE)**
Erfinder: **Dehne, Heinz-Wilhelm, Dr.**
**Krischer Strasse 81**
**W-4019 Monheim (DE)**

**EP 0 477 639 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Aminosäureamid-Derivate und ein Verfahren zu deren Herstellung, sowie deren Verwendung in Schädlingsbekämpfungsmitteln.

Die erfindungsgemäßen Substanzen besitzen eine ausgezeichnete Wirkung bei der Bekämpfung von Schädlingen. Insbesondere können die erfindungsgemäßen Substanzen als Fungizide, vor allen im Pflanzenschutz, verwendet werden.

Bestimmte Aminosäureamide sind bereits bekannt, wie beispielsweise N-tert.-Butoxycarbonyl-L-leucylbenzylamid (EP-A- 236 874, EP-A- 398 072, EP-A- 425 925).

Eine Verwendung dieser Verbindungen in Schädlingsbekämpfungsmitteln wird jedoch nicht beschrieben.

Gegenstand der vorliegenden Anmeldung sind somit neue Aminosäureamid-Derivate der allgemeinen Formel (I)

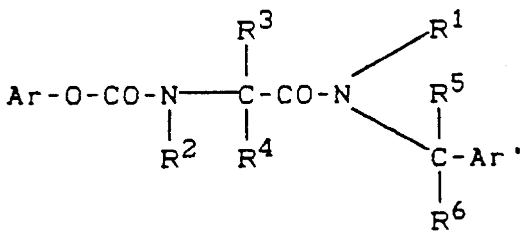

(I),

in welcher

| | |
|---|---|
| Ar und Ar' | gleich oder verschieden sind und für unsubstituiertes oder substituiertes Aryl, unsubstituiertes oder substituiertes Aralkyl, unsubstituiertes oder substituiertes Heteroaryl und unsubstituiertes oder substituiertes Heteroarylalkyl stehen, |
| $R^1$, $R^2$, $R^4$, $R^5$ und $R^6$ | gleich oder verschieden sind und für Wasserstoff oder Alkyl stehen und |
| $R^3$ | für Cycloalkyl steht. |

Die Verbindungen der Formel (I) können außerdem ein oder mehrere Chiralitätszentren enthalten und können somit in verschiedene Enantiomeren-und Diastereomerengemischen vorliegen, die gegebenenfalls in üblicher Art und Weise getrennt werden können. Sowohl die reinen Enantiomeren und Diastereomeren, als auch die Gemische werden erfindungsgemäß beansprucht.

Im folgenden wird der Einfachheit halber stets von Verbindungen der Formel (I) gesprochen, obwohl sowohl die reinen Verbindungen, als auch die Gemische mit unterschiedlichen Anteilen an isomeren, enantiomeren und diastereomeren Verbindungen gemeint sind.

Die erfindungsgemäßen substituierten Aminosäureamid-Derivate sind durch die Formel (I) allgemein definiert.

Vorzugsweise bedeutet in den allgemeinen Formeln im folgenden, falls nicht anders definiert:

Alkyl, einzeln oder in zusammengesetzten Resten

- geradkettiges oder verzweigtes Alkyl mit 1 bis 6, insbesondere 1 bis 4 Kohlenstoffatomen. Beispielhaft und vorzugsweise seien Methyl, Ethyl, n.- und i.-Propyl, n-, i-, s- und t-Butyl, genannt.

Cycloalkyl

- steht für einen 3- bis 7-gliedrigen Ring, insbesondere für einen Ring mit 3, 5 oder 6 Kohlenstoffatomen. Beispielhaft und vorzugsweise seien Cyclopropyl, Cyclobutyl, Cyclopentyl, Cyclohexyl und Cycloheptyl genannt.

Aryl

- unsubstituiertes oder substituiertes Aryl mit 6 bis 10 Kohlenstoffatomen. Beispielhaft und vorzugsweise sei jeweils unsubstituiertes oder substituiertes Phenyl und Naphthyl, insbesondere unsubstituiertes oder substituiertes Phenyl genannt.

Aralkyl

- unsubstituiertes oder substituiertes Aralkyl mit 1 bis 4, insbesondere 1 oder 2 Kohlenstoffatomen im geradkettigen oder verzweigten Alkylteil und 6 bis 10 Kohlenstoffatomen, vorzugsweise Phenyl im Arylteil. Beispielhaft und vorzugsweise seien Benzyl, 1,1- und 1,2-Phenethyl und 1,1-, 1,2-, 1,3- und 2,2-Phenylpropyl genannt.

Heteroaryl

- unsubstituierter oder substituierter 5- bis 9-gliedriger Ring, insbesondere 5- bis 7-gliedriger Ring, der 1 bis 4, bevorzugt 1 bis 3, gleiche oder verschiedene Heteroatome enthält. Als Heteroatome seien vorzugsweise Sauerstoff, Schwefel und Stickstoff genannt. Beispielhaft und vorzugsweise seien Pyrimidinyl, Pyrrolyl, Isothiazolyl, Oxazolyl, Thienyl, Furyl, Pyridazinyl, Pyrazinyl, Isooxazolyl, Thiazolyl und insbesondere Pyridyl genannt.

Heteroarylalkyl

- der Heteroarylteil entspricht den oben angegebenen Definitionen und Vorzugsbereichen. Der Alkylteil ist geradkettig oder verzweigt und enthält 1 bis 4 insbesondere 1 oder 2 Kohlenstoffatome. Beispielhaft und vorzugsweise seien Heteroarylmethyl, 1,1- und 1,2-Heteroarylethyl und 1,1-, 1,2-, 1,3- und 2,2-Heteroarylpropyl genannt.

Die gegebenenfalls substituierten Reste der allgemeinen Formeln können einen oder mehrere, vorzugsweise 1 bis 3, insbesondere 1 oder 2, gleiche oder verschiedene Substituenten tragen. Als Substituenten seien beispielhaft und vorzugsweise aufgeführt: Alkyl mit vorzugsweise 1 bis 4, insbesondere 1 oder 2 Kohlenstoffatomen, wie Methyl, Ethyl, n.- und i.-Propyl und n.-, i.-, sec.- und t.-Butyl; Alkoxy mit vorzugsweise 1 bis 4, insbesondere 1 oder 2 Kohlenstoffatomen, wie Methoxy, Ethoxy, n.- und i.-Propyloxy und n.-, i.-, sec.- und t.-Butyloxy; Alkylthio mit vorzugsweise 1 bis 4, insbesondere 1 oder 2 Kohlenstoffatomen, wie Methylthio, Ethylthio, n.- und i.-Propylthio und n.-, i.-, sec.- und t.-Butylthio; Halogenalkyl, Halogenalkoxy und Halogenalkylthio mit vorzugsweise 1 bis 4, insbesondere 1 oder 2 Kohlenstoffatomen und vorzugsweise 1 bis 9, insbesondere 1 bis 5 Halogenatomen, wobei die Halogenatome gleich oder verschieden sind und als Halogenatome, vorzugsweise Fluor, Chlor oder Brom, insbesondere Fluor stehen, wie Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Hydroxy; Halogen, vorzugsweise Fluor, Chlor, Brom und Jod, insbesondere Fluor, Chlor und Brom; Cyano; Nitro; Dialkylamino mit vorzugsweise 1 bis 4, insbesondere 1 oder 2 Kohlenstoffatomen je Alkylgruppe, wie Dimethylamino und Diethylamino; Carboxy; Alkylalkoxy mit 1 bis 4, insbesondere 1 oder 2 Kohlenstoffatomen in jedem Alkylteil; Carbonylalkoxy mit 1 bis 4, insbesondere 1 oder 2 Kohlenstoffatomen im Alkylteil, wie Carbonylmethoxy und Carbonylethoxy; Carbonylalkyl mit 1 bis 4, insbesondere 1 oder 2 Kohlenstoffatomen im Alkylteil, wie Acetyl und Propionyl; Formyl; Carbonylaryloxy mit 5 bis 10 Kohlenstoffatomen im Arylteil, wie Carbonylphenoxy; Carbonylaryl mit 6 bis 10 Kohlenstoffatomen im Arylteil, wie Benzoyl; Oxycarbonylalkyl mit 1 bis 4, insbesondere 1 oder 2 Kohlenstoffatomen im Alkylteil, wie Acetoxy; Oxycarbonylaryl mit 6 bis 10 Kohlenstoffatomen im Arylteil, wie Benzoyloxy; Carboxylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 bis 4, insbesondere 1 oder 2 Kohlenstoffatomen im Alkylteil; Sulfonamido; Sulfonalkyl; Sulfonylalkyl und Sulfonylalkoxy mit jeweils 1 bis 4, insbesondere 1 oder 2 Kohlenstoffatomen; jeweils unsubstituiertes oder durch Halogen, insbesondere Fluor, Chlor und/oder Brom substituiertes Phenyl oder Phenoxy.

Die hier aufgeführten Definitionen gelten in entsprechender Weise auch für die Definitionen in den folgenden bevorzugten Kombinationen von Resten.

Bevorzugt sind die Verbindungen der Formel (I), in welcher

| | |
|---|---|
| $R^1$, $R^2$, $R^4$, $R^5$ und $R^6$ | gleich oder verschieden sind und für Wasserstoff oder geradkettiges oder verzweigtes Alkyl mit 1 bis 4 Kohlenstoffatomen stehen, |
| $R^3$ | für einen Cycloalkylring mit 3 bis 7 Kohlenstoffatomen steht und |
| Ar und Ar' | gleich oder verschieden sind und für jeweils unsubstituiertes oder substituiertes Phenyl und Pyridyl oder unsubstituiertes oder im Phenylteil substituiertes Phenylalkyl mit 1 bis 4 Kohlenstoffatomen im geradkettigen oder verzweigten Alkylteil stehen, wobei als Substituenten im Pyridyl- bzw. Phenylteil jeweils in Frage kommen: Alkyl, Alkoxy und Alkylthio mit jeweils 1 bis 4 Kohlenstoffato- |

men; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Halogen; Cyano; Nitro; Dialkylamino mit 1 bis 4 Kohlenstoffatomen je Alkylgruppe; Carboxy; Alkylalkoxy mit 1 bis 4 Kohlenstoffatomen in jedem Alkylteil; Carbonylalkoxy mit 1 bis 4 Kohlenstoffatomen im Alkylteil; Carbonylalkyl mit 1 bis 4 Kohlenstoffatomen in Alkylteil; Formyl; Carbonylaryloxy mit 5 bis 10 Kohlenstoffatomen im Arylteil; Carbonylaryl mit 6 bis 10 Kohlenstoffatomen im Arylteil; Oxycarbonylalkyl mit 1 bis 4 Kohlenstoffatomen im Alkylteil; Oxycarbonylaryl mit 6 bis 10 Kohlenstoffatomen im Arylteil; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 bis 4 Kohlenstoffatomen im Alkylteil; Sulfonamido; Sulfonalkyl; Sulfonylalkyl und Sulfonylalkoxy mit jeweils 1 bis 4 Kohlenstoffatomen; jeweils unsubstituiertes oder durch Halogen substituiertes Phenyl oder Phenoxy.

Besonders bevorzugt sind diejenigen Verbindungen der Formel (I), in welcher

$R^1$ und $R^2$ für Wasserstoff stehen,

$R^3$ für einen Cycloalkylring mit 3 bis 6 Kohlenstoffatomen steht,

$R^4$, $R^5$ und $R^6$ gleich oder verschieden sind und für Wasserstoff, Methyl oder Ethyl stehen,

Ar für unsubstituiertes oder einfach bis dreifach, gleich oder verschieden substituiertes Phenyl steht, wobei die folgenden Substituenten in Frage kommen:
Alkyl, Alkoxy und Alkylthio mit jeweils 1 oder 2 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Hydroxy; Fluor, Chlor, Brom und Jod; Cyano; Nitro; Dialkylamino mit 1 oder 2 Kohlenstoffatomen je Alkylgruppe; Carboxy; Alkylalkoxy mit 1 oder 2 Kohlenstoffatomen in jedem Alkylteil; Carbonylalkoxy mit 1 oder 2 Kohlenstoffatomen im Alkylteil; Carbonylalkyl mit 1 oder 2 Kohlenstoffatomen im Alkylteil; Formyl; Carbonylphenoxy; Benzoyl; Oxycarbonylalkyl mit 1 oder 2 Kohlenstoffatomen; Benzoyloxy; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 oder 2 Kohlenstoffatomen im Alkylteil; Sulfonamido; Sulfonalkyl, Sulfonylalkyl und Sulfonylalkoxy mit jeweils 1 oder 2 Kohlenstoffatomen; jeweils unsubstituiertes oder durch Fluor, Chlor oder Brom substituiertes Phenyl oder Phenoxy, und

Ar' für jeweils unsubsituiertes oder einfach bis dreifach, gleich oder verschieden substituiertes Phenyl oder Pyridyl oder für unsubstituiertes oder im Phenylteil einfach bis dreifach, gleich oder verschieden substituiertes Phenylalkyl mit 1 oder 2 Kohlenstoffatomen im Alkylteil steht, wobei als Phenylsubstituenten jeweils die oben genannten Phenylsubstituenten in Frage kommen.

Ganz besonders bevorzugt sind diejenigen Verbindungen der Formel (I), in welcher

$R^1$ für Wasserstoff steht,

$R^2$ für Wasserstoff steht,

$R^4$ für Wasserstoff steht,

$R^3$ für einen Cyclopropyl-, Cyclopentyl- oder Cyclohexylring steht,

$R^5$ für Wasserstoff oder Methyl steht,

$R^6$ für Wasserstoff, Methyl oder Ethyl steht,

Ar für unsubstituiertes oder einfach bis zweifach, gleich oder verschieden substituiertes Phenyl steht, wobei die folgenden Substituenten in Frage kommen: Methyl, Ethyl, n- und i-Propyl, n-, i-, s- und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, Nitro und Cyano und

Ar' für unsubstituiertes oder im Phenylteil einfach bis zweifach, gleich oder verschieden substituiertes Benzyl oder 1,2-Phenethyl steht, insbesondere jedoch für unsubstituiertes oder einfach bis zweifach, gleich oder verschieden substituiertes Phenyl steht, wobei als Phenylsubstituenten jeweils die oben genannten Phenylsubstituenten in Frage kommen.

Man erhält die substituierten Aminosäureamid-Derivate der allgemeinen Formel (I)

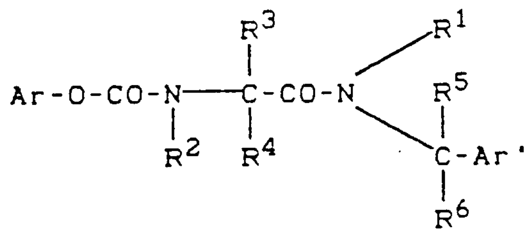

(I),

in welcher

$R^1$, $R^2$, $R^3$, $R^4$, $R^5$, $R^6$, Ar und Ar' die oben angegebene Bedeutung haben,

wenn man

eine substituierte Aminosäure der Formel (II)

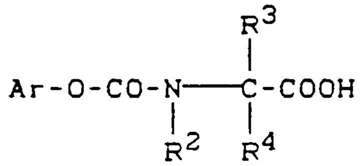

(II),

in welcher

Ar, $R^2$, $R^3$ und $R^4$ die oben angegebene Bedeutung haben, bzw. deren carboxy-aktivierte Derivate,

gegebenenfalls in Gegenwart eines Katalysators, gegebenenfalls in Gegenwart eines Säurebindemittels und gegebenenfalls in Gegenwart eines Verdünnungsmittels

mit einem Amin der Formel (III)

$HNR^1-CR^5R^6Ar'$ (III),

in welcher

Ar', $R^1$, $R^5$ und $R^6$ die oben angegebene Bedeutung haben,

umsetzt.

Verwendet man beispielsweise Phenoxycarbonyl-cyclohexylglycin und 4-Chlorphenethylamin als Ausgangsprodukte, so kann der Ablauf des erfindungsgemäßen Verfahrens durch das folgende Formelschema wiedergegeben werden:

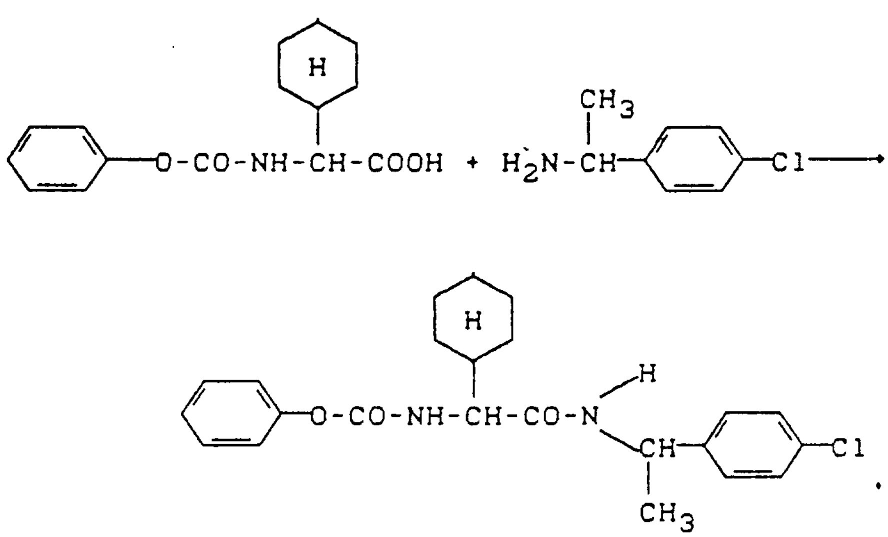

Die für die Durchführung des erfindungsgemäßen Verfahrens als Ausgangsstoffe zu verwendenden Aminosäure-Derivate sind durch die Formel (II) allgemein definiert. In dieser Formel haben Ar, $R^2$, $R^3$ und $R^4$ vorzugsweise die Bedeutungen, die bereits im Zusammenhang mit der Beschreibung der erfindungsmäßen Stoffe der Formel (I) für diese Substituenten bevorzugt genannt wurden.

Die substituierten Aminosäurederivate der Formel (II), bzw. deren carboxy-aktivierte Derivate

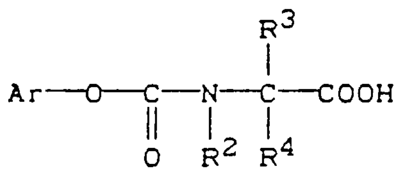

(II)

in welcher

Ar für unsubstituiertes oder substituiertes Aryl, unsubstituiertes oder substituiertes Aralkyl, unsubstituiertes oder substituiertes Heteroaryl und unsubstituiertes oder substituiertes Heteroarylalkyl steht,

$R^2$ und $R^4$ gleich oder verschieden sind und für Wasserstoff oder Alkyl stehen und

$R^3$ für Cycloalkyl steht,

sind neu, ausgenommen die Verbindungen α-(Carbonylamino)-cyclohexanessigsäure-N-benzylester (vergleiche GB 873 049); α-[[(Phenylmethoxy)carbonyl]amino]-(S)-cyclohexanessigsäure-4-nitrophenylester (vergleiche EP 19 589); α-[[(Phenylmethoxy)carbonyl]amino]-(R)-cyclohexanessigsäure-4-nitrophenylester (vergleiche EP 34 122); α-[[(Phenylmethoxy)carbonyl]amino)-(R)-cyclohexanessigsäure α-[[(Phenylmethoxy)-carbonyl]amino]-(S)-cyclohexanessigsäure (vergleiche u. a. Hoppe-Seyler's Z. Physiol. Chem., 359 (8), 897-916, 1978) und α-(Carboxyamino)-(L)-cyclohexanessigsäure-N-benzylester (vergleiche J. Med. Chem., 12 (5), 737-740, 1969).

Die neuen substituierten Aminosäurederivate der Formel (II), bzw. deren carboxy-aktivierte Ester lassen sich nach bekannten Verfahen in analoger Weise erhalten, indem man beispielsweise Aminosäurederivate der Formel (IV)

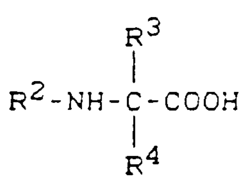

(IV)

in welcher

$R^2$, $R^3$ und $R^4$ die oben angegebene Bedeutung haben,

mit Säurechloriden der Formel (V)

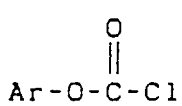

(V)

in welcher

Ar die oben angegebene Bedeutung hat,

gegebenenfalls in Gegenwart eines Säurebindemittels, wie beispielsweise Natronlauge oder Pyridin und gegebenenfalls eines Verdünnungsmittels, wie beispielsweise Dichlormethan, bei Temperaturen von -10 °C bis +10 °C umsetzt (vergleiche u. a. Tetrahedron Lett. 30 (39), 5227-5230, 1989 und J. Med. Chem., 12 (5), 737-740, 1969).

Die zur Herstellung der substituierten Aminosäurederivate der Formel (II) als Ausgangsstoffe benötigten Aminosäurederivate der Formel (IV) sind durch die Formel (IV) allgemein definiert. In dieser Formel (IV) stehen $R^2$, $R^3$ und $R^4$ für diejenigen Reste, die bereits im Zusammenhang mit der Beschreibung der erfindungsgemäßen Verbindung der Formel (I) für diese Substituenten genannt wurden.

Die Aminosäurederivate der Formel (IV) sind allgemein bekannte Verbindungen der organischen Chemie.

In den zur Herstellung der substituierten Aminosäurederivate der Formel (II) weiterhin als Ausgangsstoffe benötigten Säurechloriden der Formel (V) hat Ar die bereits im Zusammenhang mit der Beschreibung der erfindungsgemäßen Verbindung der Formel (I) angegebene Bedeutung.
Die Säurechloride der Formel (V) sind ebenfalls allgemein bekannte Verbindungen der organischen Chemie.

Als carboxy-aktivierte Derivate der Aminosäuren der Formel (II) kommen alle Carboxy-aktivierten Derivate in Frage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxysuccinimidester sowie mit Kondensationsmitteln, wie z.B. Dicyclohexylcarbodiimid oder Carbonyldiimidazol, in situ erzeugte aktivierte Formen der Aminosäuren.

Vorzugsweise werden die den Aminosäuren der Formel (II) entsprechenden Säurechloride und gemischten Anhydride eingesetzt. Sie können hergestellt werden, indem man die Aminosäuren der Formel (II) oder deren Salze mit einem Halogenierungsmittel oder einem der allgemein bekannten Mittel zur Herstellung von gemischten Anhydriden, wie beispielsweise Phosphorpentachlorid, Thionylchlorid, Oxalylchlorid oder Chlorameisensäureisobutylester, in allgemein bekannter Art und Weise umsetzt. Bevorzugt ist der Einsatz von Chlorameisensäureisobutylester.

Die Umsetzung kann in Gegenwart indifferenter Verdünnungsmittel wie z.B. aromatische, nichtaromatische oder halogenierte Kohlenwasserstoffe wie: Ketone, wie z.B. Aceton; Ester, wie z.B. Ethylacetat; Amide, wie z.B. Dimethylformamid-; Nitrile, wie z.B. Acetonitril; Chlorkohlenwasserstoffe, wie z.B. Methylenchlorid; Kohlenwasserstoffe, wie z.B. Toluol; oder Ether, wie z.B. Tetrahydrofuran bzw. deren Mischungen und/oder in Gegenwart eines Säurebindemittels, wie vorzugsweise eines tertiären Amins, wie z.B. Triethylamin, Pyridin oder N-Methylpiperidin, bei Temperaturen von -78 °C bis 100 °C, vorzugsweise -60 °C bis 25 °C, durchgeführt werden.
Die außerdem für die Durchführung des erfindungsgemäßen Verfahrens als Ausgangsstoffe zu verwendenden Amine sind durch die Formel (III) allgemein definiert. In diesen Formeln haben $R^1$, Ar', $R^5$ und $R^6$ die oben genannten Bedeutungen.

Die Amine der Formel (III) sind allgemein bekannte Verbindungen der organischen Chemie.

Als Verdünnungsmittel kommen für das erfindungsgemäße Verfahren inerte, organische Lösungsmittel wie: Ketone, wie Aceton oder Ethylmethylketon; Ester wie Ethyl- oder Methylacetat; Amide wie Dimethylformamid; Nitrile, wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren Mischungen in Frage.

Als Säurebindemittel kommen für das erfindungsgemäße Verfahren übliche anorganische und organische Säurebinder in Frage. Hierzu gehören vorzugsweise tertiäre Amine, wie Triethylamin, Pyridin oder N-Methylpiperidin, sowie anorganische Basen, z.B. Metallhydroxide wie Natrium- und Kaliumhydroxid oder Metallcarbonate wie Natriumcarbonat oder Calciumcarbonat.

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfahrens in einem größeren Bereich variiert werden. Im allgemeinen arbeitet man bei -78 bis +120 °C, vorzugsweise bei -60 bis +40 °C.

Bei der Durchführung des erfindungsgemäßen Verfahrens arbeitet man vorzugsweise in äquimolaren Mengen.

Dabei werden die Aminosäurederivate der Formel (II) als reine optische Isomere (D bzw. L-Form) oder als Racemate eingesetzt.

Die Erfindung umfaßt sowohl die reinen Isomeren als auch die Gemische. Diese Gemische können nach gebräuchlichen Methoden, z.B. selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so z.B. durch Salzbildung mit optisch aktiven Säuren wie Champhersulfonsäure oder Dibenzoylweinsäure und selektive Kristallisation oder durch Derivatisierung mit geeigneten, optisch aktiven Reagenzien, Trennung der diastereomeren Derivate und Rückspaltung oder Trennung an optisch aktivem Säulenmaterial.

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Phytophthora-Arten an Tomaten einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächen-aktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage; z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

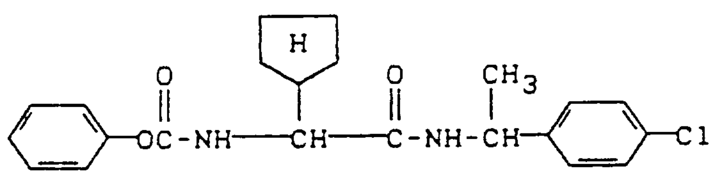

1,47 g (6 mmol) Phenoxycarbonyl-L-cyclopentylglycin werden in 50 ml $CH_2Cl_2$ gelöst und bei -20 °C mit 0,59 g (6 mmol) N-Methylpiperdidin versetzt. Nach 5 Minuten Rühren bei -20 °C läßt man 0,82 g (6 mmol) Chlorameisensäureisobutylester zutropfen, rührt 10 Minuten bei -20 °C nach, kühlt auf -60 °C ab und gibt 0,93 g (6 mmol) 1-(4-Chlorphenyl)ethylamin gelöst in 5 ml $CH_2Cl_2$ zu. Es wird 2 Stunden bei -15 °C nachgerührt und danach weitere 15 Stunden bei Raumtemperatur. Zur Aufarbeitung wird abfiltriert, die Lösung im Vakuum eingeengt und der Rückstand in $CH_2Cl_2$ aufgenommen. Die organische Phase wird nacheinander mit Wasser, $NaHCO_3$-Lösung und Wasser gewaschen, über $Na_2SO_4$ getrocknet und im Vakuum vom Lösungsmittel befreit. Man erhält 1,05 g (44% d.Th.) farbloses $N^2$-Phenoxycarbonyl-$N^1$-[rac.-1-(4-chlorphenyl)ethyl]-L-cyclopentylglycin mit einem Schmelzpunkt von 167 °C.

Analog Beispiel 1 werden die folgenden Verbindungen der Formel (I) erhalten:

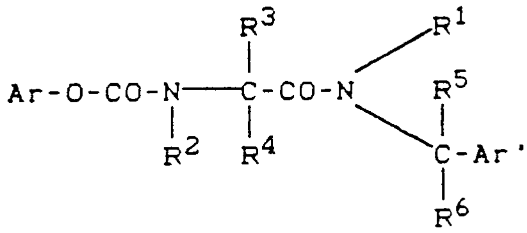

Tabelle 1

| Bsp. Nr. | Ar | $R^1$ | $R^2$ | $R^3$ | $R^4$ | $R^5$ | $R^6$ | Ar' | physik. Konst. |
|---|---|---|---|---|---|---|---|---|---|
| 2 | 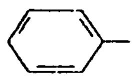 | H | H | 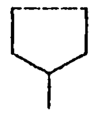 | H | H | $CH_3$ | 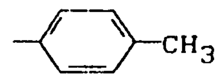 | F. 160° C Rac.Amid d. L-Aminosäure |
| 3 | 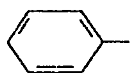 | H | H | 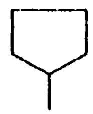 | H | H | $CH_3$ | 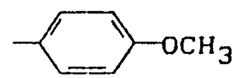 | F. 146° C Rac.Amid d. L-Aminosäure |
| 4 | 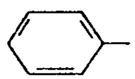 | H | H | 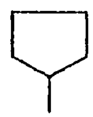 | H | H | $CH_3$ | 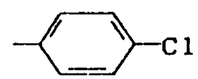 | F.187° C (R+)Amid d. L-Aminosäure |

Beispiel A

Phytophthora-Test (Tomate) /protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Die Verbindungen gemäß den Herstellungsbeispielen (1), (2), (3) und (4) zeigen eine ausgezeichnete fungizide Wirksamkeit.

**Patentansprüche**

**1.** Aminosäureamid-Derivate der allgemeinen Formel (I)

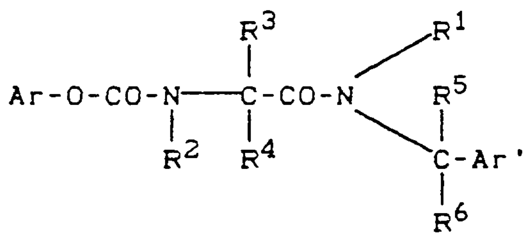

(I),

in welcher

| | |
|---|---|
| Ar und Ar' | gleich oder verschieden sind und für unsubstituiertes oder substituiertes Aryl, unsubstituiertes oder substituiertes Aralkyl, unsubstituiertes oder substituiertes Heteroaryl und unsubstituiertes oder substituiertes Heteroarylalkyl stehen, |
| $R^1$, $R^2$, $R^4$, $R^5$ und $R^6$ | gleich oder verschieden sind und für Wasserstoff oder Alkyl stehen und |
| $R^3$ | für Cycloalkyl steht. |

**2.** Verbindungen der Formel (I) gemäß Anspruch 1, in welcher

| | |
|---|---|
| $R^1$, $R^2$, $R^4$, $R^5$ und $R^6$ | gleich oder verschieden sind und für Wasserstoff oder geradkettiges oder verzweigtes Alkyl mit 1 bis 4 Kohlenstoffatomen stehen, |
| $R^3$ | für einen Cycloalkylring mit 3 bis 7 Kohlenstoffatomen steht und |
| Ar und Ar' | gleich oder verschieden sind und für jeweils unsubstituiertes oder substituiertes Phenyl und Pyridyl oder unsubstituiertes oder im Phenylteil substituiertes Phenylalkyl mit 1 bis 4 Kohlenstoffatomen im geradkettigen oder verzweigten Alkylteil stehen, wobei als Substituenten im Pyridyl- bzw. Phenylteil jeweils in Frage kommen: Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Halogen; Cyano; Nitro; Dialkylamino mit 1 bis 4 Kohlenstoffatomen je Alkylgruppe; Carboxy; Alkylalkoxy mit 1 bis 4 Kohlenstoffatomen in jedem Alkylteil; Carbonylalkoxy mit 1 bis 4 Kohlenstoffatomen im Alkylteil; Carbonylalkyl mit 1 bis 4 Kohlenstoffatomen in Alkylteil; Formyl; Carbonylaryloxy mit 5 bis 10 Kohlenstoffatomen im Arylteil; Carbonylaryl mit 6 bis 10 Kohlenstoffatomen im Arylteil; Oxycar- |

bonylalkyl mit 1 bis 4 Kohlenstoffatomen im Alkylteil; Oxycarbonylaryl mit 6 bis 10 Kohlenstoffatomen im Arylteil; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 bis 4 Kohlenstoffatomen im Alkylteil; Sulfonamido; Sulfonalkyl; Sulfonylalkyl und Sulfonylalkoxy mit jeweils 1 bis 4 Kohlenstoffatomen; jeweils unsubstituiertes oder durch Halogen substituiertes Phenyl oder Phenoxy.

**3.** Verbindungen der Formel (I) gemäß Anspruch 1, in welcher

$R^1$ und $R^2$ für Wasserstoff stehen,

$R^3$ für einen Cycloalkylring mit 3 bis 6 Kohlenstoffatomen steht,

$R^4$, $R^5$ und $R^6$ gleich oder verschieden sind und für Wasserstoff, Methyl oder Ethyl stehen,

Ar für unsubstituiertes oder einfach bis dreifach, gleich oder verschieden substituiertes Phenyl steht, wobei die folgenden Substituenten in Frage kommen: Alkyl, Alkoxy und Alkylthio mit jeweils 1 oder 2 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Hydroxy; Fluor, Chlor, Brom und Jod; Cyano; Nitro; Dialkylamino mit 1 oder 2 Kohlenstoffatomen je Alkylgruppe; Carboxy; Alkylalkoxy mit 1 oder 2 Kohlenstoffatomen in jedem Alkylteil; Carbonylalkoxy mit 1 oder 2 Kohlenstoffatomen im Alkylteil; Carbonylalkyl mit 1 oder 2 Kohlenstoffatomen im Alkylteil; Formyl; Carbonylphenoxy; Benzoyl; Oxycarbonylalkyl mit 1 oder 2 Kohlenstoffatomen; Benzoyloxy; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 oder 2 Kohlenstoffatomen im Alkylteil; Sulfonamido; Sulfonalkyl, Sulfonylalkyl und Sulfonylalkoxy mit jeweils 1 oder 2 Kohlenstoffatomen; jeweils unsubstituiertes oder durch Fluor, Chlor oder Brom substituiertes Phenyl oder Phenoxy, und

Ar' für jeweils unsubsituiertes oder einfach bis dreifach, gleich oder verschieden substituiertes Phenyl oder Pyridyl oder für unsubstituiertes oder im Phenylteil einfach bis dreifach, gleich oder verschieden substituiertes Phenylalkyl mit 1 oder 2 Kohlenstoffatomen im Alkylteil steht, wobei als Phenylsubstituenten jeweils die oben genannten Phenylsubstituenten in Frage kommen.

**4.** Verbindungen der Formel (I) gemäß Anspruch 1, in welcher

$R^1$ für Wasserstoff steht,

$R^2$ für Wasserstoff steht,

$R^4$ für Wasserstoff steht,

$R^3$ für einen Cyclopropyl-, Cyclopentyl- oder Cyclohexylring steht,

$R^5$ für Wasserstoff oder Methyl steht,

$R^6$ für Wasserstoff, Methyl oder Ethyl steht,

Ar für unsubstituiertes oder einfach bis zweifach, gleich oder verschieden substituiertes Phenyl steht, wobei die folgenden Substituenten in Frage kommen: Methyl, Ethyl, n- und i-Propyl, n-, i-, s-und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, Nitro und Cyano und

Ar' für unsubstituiertes oder im Phenylteil einfach bis zweifach, gleich oder verschieden substituiertes Benzyl oder 1,2-Phenethyl steht, insbesondere jedoch für unsubstituiertes oder einfach bis zweifach, gleich oder verschieden substituiertes Phenyl steht, wobei als Phenylsubstituenten jeweils die oben genannten Phenylsubstituenten in Frage kommen.

5. Verfahren zur Herstellung von Aminosäureamid-Derivaten der allgemeinen Formel (I)

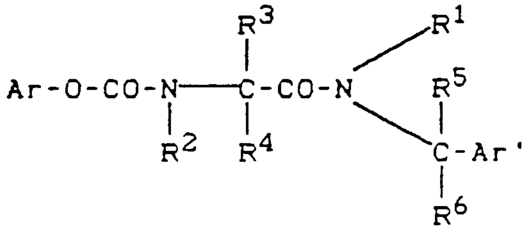

(I),

in welcher
$R^1$, $R^2$, $R^3$, $R^4$, $R^5$, $R^6$, Ar und Ar' die in Anspruch 1 angegebene Bedeutung haben,
dadurch gekennzeichnet, daß man
eine substituierte Aminosäure der Formel (II)

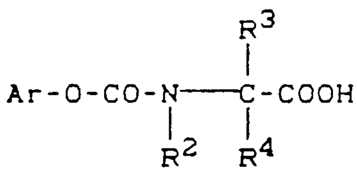

(II),

in welcher
Ar, $R^2$, $R^3$ und $R^4$ die oben angegebene Bedeutung haben, bzw. deren carboxy-aktivierte Derivate,
gegebenenfalls in Gegenwart eines Katalysators, gegebenenfalls in Gegenwart eines Säurebindemittels und gegebenenfalls in Gegenwart eines Verdünnungsmittels
mit einem Amin der Formel (III)

$HNR^1-CR^5R^6Ar'$ (III),

in welcher
Ar', $R^1$, $R^5$ und $R^6$ die oben angegebene Bedeutung haben,
umsetzt.

6. Schädlingsbekämpfungsmittel, gekennzeichnet durch einen Gehalt an mindestens einen Aminosäureamid-Derivat der Formel (I) nach den Ansprüchen 1 bis 5.

7. Verwendung von Aminosäureamid-Derivaten der Formel (I) nach den Ansprüchen 1 bis 5 zur Bekämpfung von Schädlingen.

8. Verfahren zur Bekämpfung von Schädlingen, dadurch gekennzeichnet, daß man Aminosäureamid-Derivate der Formel (I) nach den Ansprüchen 1 bis 5 auf Schädlinge und/oder ihren Lebensraum einwirken läßt.

9. Verfahren zur Herstellung von Schädlingsbekämpfungsmitteln, dadurch gekennzeichnet, daß man Aminosäureamid-Derivate der Formel (I) nach den Ansprüchen 1 bis 5 mit Streckmitteln und/oder oberflächenaktiven Mitteln vermischt.

**10.** Aminosäurederivate der Formel (II), bzw. deren carboxy-aktivierte Derivate

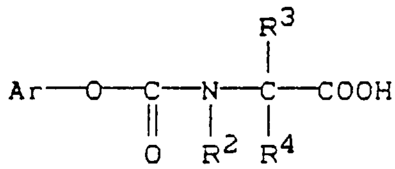

(II)

in welcher

Ar für unsubstituiertes oder substituiertes Aryl, unsubstituiertes oder substituiertes Aralkyl, unsubstituiertes oder substituiertes Heteroaryl und unsubstituiertes oder substituiertes Heteroarylalkyl steht,

$R^2$ und $R^4$ gleich oder verschieden sind und für Wasserstoff oder Alkyl stehen und

$R^3$ für Cycloalkyl steht,

ausgenommen die Verbindungen α-(Carbonylamino)cyclohexanessigsäure-N-benzylester, α-[[-(Phenylmethoxy)carbonyl]amino]-(S)-cyclohexanessigsäure-4-nitrophenylester, α-[[(Phenylmethoxy)-carbonyl]amino]-(R)-cyclohexanessigsäure-4-nitrophenylester, α-[[(Phenylmethoxy)carbonyl]amino]-(R)-cyclohexanessigsäure α-[[(Phenylmethoxy)carbonyl]amino]-(S)-cyclohexanessigsäure und α-(Carboxyamino)-(L)-cyclohexanessigsäure-N-benzylester.

**11.** Verfahren zur Herstellung von Aminosäurederivate der Formel (II), bzw. deren carboxy-aktivierte Ester gemäß Anspruch 10, dadurch gekennzeichnet, daß man Aminosäurederivate der Formel (IV)

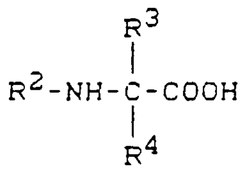

(IV)

in welcher

$R^2$, $R^3$ und $R^4$ die in Anspruch 1 angegebene Bedeutung haben,

mit Säurechloriden der Formel (V)

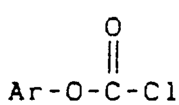

(V)

in welcher

Ar die in Anspruch 10 angegebene Bedeutung hat,

gegebenenfalls in Gegenwart eines Säurebindemittels, und gegebenenfalls eines Verdünnungsmittels, bei Temperaturen von -10 °C bis +10 °C umsetzt.

## Claims

**1.** Amino acid amide derivatives of the general formula (I)

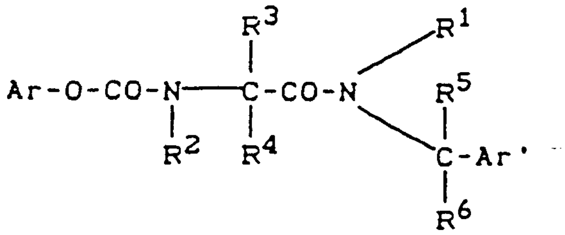

(I)

in which

| | |
|---|---|
| Ar and Ar' | are identical or different and represent unsubstituted or substituted aryl, unsubstituted or substituted aralkyl, unsubstituted or substituted heteroaryl and unsubstituted or substituted heteroarylalkyl, |
| $R^1$, $R^2$, $R^4$, $R^5$ and $R^6$ | are identical or different and represent hydrogen or alkyl and |
| $R^3$ | represents cycloalkyl. |

**2.** Compounds of the formula (I) according to Claim 1, in which

| | |
|---|---|
| $R^1$, $R^2$, $R^4$, $R^5$ and $R^6$ | are identical or different and represent hydrogen or straight-chain or branched alkyl having 1 to 4 carbon atoms, |
| $R^3$ | represents a cycloalkyl ring having 3 to 7 carbon atoms and |
| Ar and Ar' | are identical or different and represent in each case unsubstituted or substituted phenyl and pyridyl or phenylalkyl which has 1 to 4 carbon atoms in the straight-chain or branched alkyl moiety and which is unsubstituted or substituted in the phenyl moiety, suitable substituents in the pyridyl or phenyl moiety in each case being: alkyl, alkoxy and alkylthio, each of which has 1 to 4 carbon atoms; halogenoalkyl, halogenoalkoxy and halogenoalkylthio, each of which has 1 to 4 carbon atoms and 1 to 9 halogen atoms, the halogen atoms being identical or different; hydroxyl; halogen; cyano; nitro; dialkylamino having 1 to 4 carbon atoms per alkyl group; carboxyl; alkylalkoxy having 1 to 4 carbon atoms in each alkyl moiety; carbonylalkoxy having 1 to 4 carbon atoms in the alkyl moiety; carbonylalkyl having 1 to 4 carbon atoms in the alkyl moiety; formyl; carbonylaryloxy having 5 to 10 carbon atoms in the aryl moiety; carbonylaryl having 6 to 10 carbon atoms in the aryl moiety; oxycarbonylalkyl having 1 to 4 carbon atoms in the alkyl moiety; oxycarbonylaryl having 6 to 10 carbon atoms in the aryl moiety; carbonylamino, carbonylaminoalkyl, carbonylaminodialkyl, aminocarbonyl, alkylaminocarbonyl, aminocarbonylalkyl and alkylaminocarbonylalkyl, each of which has 1 to 4 carbon atoms in the alkyl moiety; sulphonamido; sulphonalkyl; sulphonylalkyl and sulphonylalkoxy, each of which has 1 to 4 carbon atoms; and phenyl or phenoxy, each of which is unsubstituted or substituted by halogen. |

**3.** Compounds of the formula (I) according to Claim 1, in which

| | |
|---|---|
| $R^1$ and $R^2$ | represent hydrogen, |
| $R^3$ | represents a cycloalkyl ring having 3 to 6 carbon atoms, |
| $R^4$, $R^5$ and $R^6$ | are identical or different and repre sent hydrogen, methyl or ethyl, |
| Ar | represents phenyl which is unsubstituted or monosubstituted to trisubstituted by identical or different substituents, suitable substituents being the following: alkyl, alkoxy and alkylthio, each of which has 1 or 2 carbon atoms; halogenoalkyl, halogenoalkoxy and halogenoalkylthio, each of which has 1 or 2 carbon atoms and 1 to 5 halogen atoms, the halogen atoms being identical of different; hydroxyl; fluorine, chlorine, bromine and iodine; cyano; nitro; dialkylamino having |

1 or 2 carbon atoms per alkyl group; carboxyl; alkylalkoxy having 1 or 2 carbon atoms in each alkyl moiety; carbonylalkoxy having 1 or 2 carbon atoms in the alkyl moiety; carbonylalkyl having 1 or 2 carbon atoms in the alkyl moiety; formyl; carbonylphenoxy; benzoyl; oxycarbonylalkyl having 1 or 2 carbon atoms; benzoyloxy; carbonylamino, carbonylaminoalkyl, carbonylaminodialkyl, aminocarbonyl, alkylaminocarbonyl, aminocarbonylalkyl and alkylaminocarbonylalkyl, each of which has 1 or 2 carbon atoms in the alkyl moiety; sulphonamido; sulphonalkyl, sulphonylalkyl and sulphonylalkoxy, each of which has 1 or 2 carbon atoms; or phenyl or phenoxy, each of which is unsubstituted or substituted by fluorine, chlorine or bromine, and

Ar' represents phenyl or pyridyl, each of which is unsubstituted or monosubstituted to trisubstituted by identical or different substituents, or represents phenylalkyl which has 1 or 2 carbon atoms in the alkyl moiety and which is unsubstituted or monosubstituted to trisubstituted in the phenyl moiety by identical or different substituents, suitable phenyl substituents in each case being the phenyl substituents mentioned above.

**4.** Compounds of the formula (I) according to Claim 1, in which

$R^1$ represents hydrogen,
$R^2$ represents hydrogen,
$R^4$ represents hydrogen,
$R^3$ represents a cyclopropyl, cyclopentyl or cyclohexyl ring,
$R^5$ represents hydrogen or methyl,
$R^6$ represents hydrogen, methyl or ethyl,
Ar represents phenyl which is unsubstituted or monosubstituted or disubstituted by identical or different substituents, suitable substituents being the following: methyl, ethyl, n- and i-propyl, n-, i-, s- and t-butyl, methoxy, ethoxy, i- and n-propoxy, n-, i-, s-and t-butoxy, trifluoromethyl, difluoromethyl, pentafluoroethyl, tetrafluoroethyl, trifluorochloroethyl, trifluoroethyl, trifluoroethoxy, difluoromethoxy, pentafluoroethoxy, tetrafluoroethoxy, trifluorochloroethoxy, trifluoromethoxy and trifluoromethylthio; chlorine, bromine, fluorine, nitro and cyano and
Ar' represents benzyl or 1,2-phenethyl, each of which is unsubstituted or monosubstituted or disubstituted in the phenyl moiety by identical or different substituents, but in particular represents phenyl which is unsubstituted or monosubstituted or disubstituted by identical or different substituents, suitable phenyl substituents in each case being the phenyl substituents mentioned above.

**5.** Process for the preparation of amino acid amide derivatives of the general formula (I)

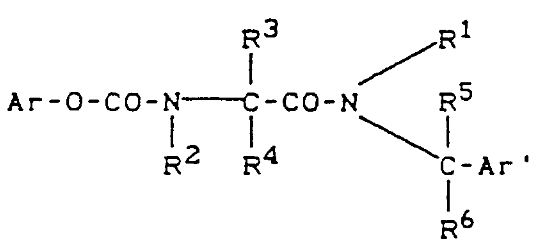

(I)

in which

$R^1$, $R^2$, $R^3$, $R^4$, $R^5$, $R^6$, Ar and Ar' have the meaning given in Claim 1,

characterised in that

a substituted amino acid of the formula (II)

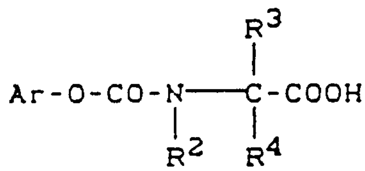

(II)

in which

Ar, $R^2$, $R^3$ and $R^4$ have the abovementioned meaning, or their carboxyl-activated derivatives,

are reacted with an amine of the formula (III)

$HNR^1\text{-}CR^5R^6Ar'$ (III)

in which

Ar', $R^1$, $R^5$ and $R^6$ have the abovementioned meaning,

if appropriate in the presence of a catalyst, if appropriate in the presence of an acid-binding agent and if appropriate in the presence of a diluent.

**6.** Pesticide, characterised in that it contains at least one amino acid amide derivative of the formula (I) according to Claims 1 to 5.

**7.** Use of amino acid amide derivatives of the formula (I) according to Claims 1 to 5 for combating pests.

**8.** Method of combating pests, characterised in that amino acid amide derivatives of the formula (I) according to Claims 1 to 5 are allowed to act on pests and/or their environment.

**9.** Process for the preparation of pesticides, characterised in that amino acid amide derivatives of the formula (I) according to Claims 1 to 5 are mixed with extenders and/or surface-active agents.

**10.** Amino acid derivatives of the formula (II), or their carboxyl-activated derivatives,

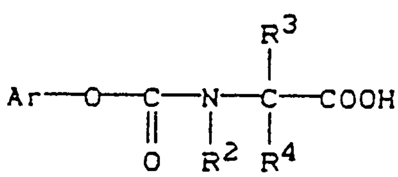

(II)

in which

Ar represents unsubstituted or substituted aryl, unsubstituted or substituted aralkyl, unsubstituted or substituted heteroaryl and unsubstituted or substituted heteroarylalkyl,

$R^2$ and $R^4$ are identical or different and represent hydrogen or alkyl and

$R^3$ represents cycloalkyl,

with the exception of the compounds N-benzyl α-(carbonylamino)cyclohexaneacetate, 4-nitrophenyl α-[[(phenylmethoxy)carbonyl]amino]-(S)-cyclohexaneacetate,4-nitrophenyl α-[[(phenylmethoxy)carbonyl]-amino]-(R)-cyclohexaneacetate, α-[[(phenylmethoxy)carbonyl]amino]-(R)-cyclohexaneacetic acid, α-[[(phenylmethoxy)carbonyl]amino]-(S)-cyclohexaneacetic acid and N-benzyl α-(carboxyamino)-(L)-cyclohexaneacetate.

**11.** Process for the preparation of amino acid derivatives of the formula (II) or their carboxyl-activated esters according to Claim 10, characterised in that amino acid derivatives of the formula (IV)

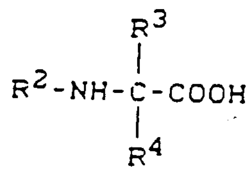

(IV)

in which

$R^2$, $R^3$ and $R^4$ have the meaning given in Claim 1,

are reacted with acid chlorides of the formula (V)

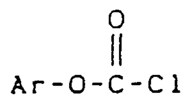

(V)

in which

Ar has the meaning given in Claim 10,

if appropriate in the presence of an acid-binding agent and if appropriate in the presence of a diluent, at temperatures from -10 °C to +10 °C.

## Revendications

**1.** Dérivés d'amides d'amino-acides de formule générale (I)

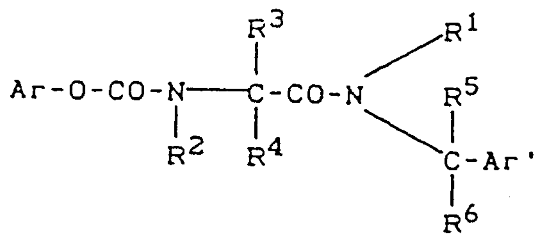

(I)

dans laquelle

| | |
|---|---|
| Ar et Ar' | sont identiques ou différents et représentent un groupe aryle non substitué ou substitué, un groupe aralkyle non substitué ou substitué, un groupe hétéroaryle non substitué ou substitué et un groupe hétéroarylalkyle non substitué ou substitué, |
| $R^1$, $R^2$, $R^4$ $R^5$ et $R^6$ | sont identiques ou différents et représentent de l'hydrogène ou un groupe alkyle et |
| $R^3$ | est un groupe cycloalkyle. |

**2.** Composés de formule (I) suivant la revendication 1, dans laquelle

| | |
|---|---|
| $R^1$, $R^2$, $R^4$ $R^5$ et $R^6$ | sont identiques ou différents et représentent de l'hydrogène ou un groupe alkyle linéaire ou ramifié ayant 1 à 4 atomes de carbone, |
| $R^3$ | est un noyau cycloalkyle de 3 à 7 atomes de carbone et |
| Ar et Ar' | sont identiques ou différents et représentent un groupe phényle et un groupe pyridyle, chacun étant non substitué ou substitué, ou un groupe phénylalkyle non substitué ou substitué dans la partie phényle ayant 1 à 4 atomes de carbone dans la partie alkyle linéaire ou ramifiée, en considérant comme substituants de chacune des parties pyridyle et phényle : des substituants alkyle, alkoxy et alkylthio ayant chacun 1 à 4 atomes de carbone ; halogénalkyle, halogénalkoxy et halogénalkylthio ayant chacun 1 à 4 atomes de carbone et 1 à 9 atomes d'halogènes, les atomes d'halogènes |

étant identiques ou différents ; un substituant hydroxy ; halogéno ; cyano ; nitro ; dialkylamino ayant 1 à 4 atomes de carbone par groupe alkyle ; carboxy ; alkylalkoxy ayant 1 à 4 atomes de carbone dans chaque partie alkyle ; carbonylalkoxy ayant 1 à 4 atomes de carbone dans la partie alkyle ; carbonylalkyle ayant 1 à 4 atomes de carbone dans la partie alkyle ; formyle ; carbonylaryloxy ayant 5 à 10 atomes de carbone dans la partie aryle ; carbonylaryle ayant 6 à 10 atomes de carbone dans la partie aryle ; oxycarbonylalkyle ayant 1 à 4 atomes de carbone dans la partie alkyle ; oxycarbonylaryle ayant 6 à 10 atomes de carbone dans la partie aryle ; carbonylamino, carbonylaminoalkyle, carbonylaminodialkyle, aminocarbonyle, alkylaminocarbonyle, aminocarbonylalkyle et alkylaminocarbonylalkyle ayant chacun 1 à 4 atomes de carbone dans la partie alkyle ; sulfonamido ; sulfonalkyle ; sulfonylalkyle et sulfonylalkoxy ayant chacun 1 à 4 atomes de carbone ; phényle ou phénoxy non substitué ou substitué chacun par un halogène.

**3.** Composés de formule (I) suivant la revendication 1, dans laquelle

$R^1$ et $R^2$ représentent de l'hydrogène,

$R^3$ est un noyau cycloalkyle de 3 à 6 atomes de carbone,

$R^4$, $R^5$ et $R^6$ sont identiques ou différents et représentent de l'hydrogène, un groupe méthyle ou éthyle,

Ar est un groupe phényle non substitué ou portant 1 à 3 substituants identiques ou différents, en considérant les substituants suivants :
alkyle, alkoxy et alkylthio ayant chacun 1 ou 2 atomes de carbone ; halogénalkyle, halogénalkoxy et halogénalkylthio ayant 1 ou 2 atomes de carbone et 1 à 5 atomes d'halogènes, les atomes d'halogènes étant identiques ou différents ; hydroxy ; fluoro, chloro, bromo et iodo ; cyano ; nitro ; dialkylamino ayant 1 ou 2 atomes de carbone par groupe alkyle ; carboxy ; alkylalkoxy ayant 1 ou 2 atomes de carbone dans chaque partie alkyle ; carbonylalkoxy ayant 1 ou 2 atomes de carbone dans la partie alkyle ; carbonylalkyle ayant 1 ou 2 atomes de carbone dans la partie alkyle ; formyle ; carbonylphénoxy ; benzoyle ; oxycarbonylalkyle ayant 1 ou 2 atomes de carbone ; benzoyloxy ; carbonylamino, carbonylaminoalkyle, carbonylaminodialkyle, aminocarbonyle, alkylaminocarbonyle, aminocarbonylalkyle et alkylaminocarbonylalkyle ayant chacun 1 ou 2 atomes de carbone dans la partie alkyle ; sulfonamido ; sulfonalkyle, sulfonylalkyle et sulfonylalkoxy ayant chacun 1 ou 2 atomes de carbone ; phényle ou phénoxy non substitué ou substitué par du fluor, du chlore ou du brome, et

Ar' est un groupe phényle ou un groupe pyridyle non substitué ou portant chacun 1 à 3 substituants identiques ou différents, ou un groupe phénylalkyle ayant 1 ou 2 atomes de carbone dans la partie alkyle, non substitué ou portant dans la partie phényle un à trois substituants identiques ou différents, en considérant comme substituant de la partie phényle chacun des substituants mentionnés ci-dessus pour un groupe phényle.

**4.** Composés de formule (I) suivant la revendication 1, dans laquelle

$R^1$ représente de l'hydrogène,

$R^2$ est de l'hydrogène,

$R^4$ est de l'hydrogène,

$R^3$ est un noyau cyclopropyle, cyclopentyle ou cyclohexyle,

$R^5$ est de l'hydrogène ou un groupe méthyle,

$R^6$ est de l'hydrogène, un groupe méthyle ou éthyle,

Ar est un groupe phényle non substitué ou portant 1 ou 2 substituants identiques ou différents, en considérant les substituants suivants : méthyle, éthyle, n-propyle, isopropyle, n-butyle, isobutyle, sec-butyle, tertiobutyle, méthoxy, éthoxy, isopropoxy, n-propoxy, n-butoxy, isobutoxy, sec.-butoxy, tertiobutoxy, trifluorométhyle, difluorométhyle, pentafluoréthyle, tétrafluoréthyle, trifluorochloréthyle, trifluoréthyle, trifluoréthoxy, difluorométhoxy, pentafluoréthoxy, tétrafluoréthoxy, trifluorochloréthoxy, trifluorométhoxy et trifluorométhylthio ; chlore, brome, fluor, nitro et cyano et

Ar' est un groupe benzyle ou 1,2-phénéthyle non substitué ou portant dans la partie phényle un ou deux substituants identiques ou différents, en particulier toutefois un groupe phényle non substitué ou portant un ou deux substituants identiques ou différents, en considérant comme substituants du groupe phényle chacun des substituants mentionnés ci-dessus pour un groupe phényle.

**5.** Procédé de production de dérivés d'amides d'amino-acides de formule générale (I)

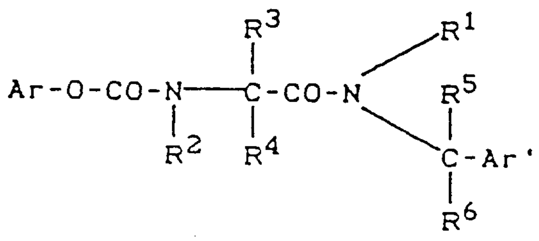 (I)

dans laquelle
$R^1$, $R^2$, $R^3$, $R^4$, $R^5$, $R^6$, Ar et Ar' ont la définition indiquée dans la revendication 1,
caractérisé en ce qu'on fait réagir un amino-acide substitué de formule (II)

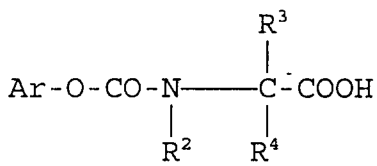 (II)

dans laquelle
Ar, $R^2$, $R^3$ et $R^4$ ont la définition indiquée ci-dessus, ou leurs dérivés à groupes carboxy activés, le cas échéant en présence d'un catalyseur, éventuellement en présence d'un accepteur d'acide et en la présence éventuelle d'un diluant,
avec une amine de formule (III),

$HNR^1\text{-}CR^5R^6Ar'$ (III),

dans laquelle
Ar', $R^1$, $R^5$ et $R^6$ ont la définition indiquée ci-dessus.

**6.** Compositions pesticides, caractérisées par une teneur en au moins un dérivé d'amides d'amino-acides de formule (I) suivant les revendications 1 à 5.

**7.** Utilisation de dérivés d'amides d'amino-acides de formule (I) suivant les revendications 1 à 5 pour combattre des parasites.

**8.** Procédé pour combattre des parasites, caractérisé en ce qu'on fait agir des dérivés d'amides d'amino-acides de formule (I) suivant les revendications 1 à 5 sur les parasites et/ou sur leur milieu.

**9.** Procédé de préparation de compositions pesticides, caractérisé en ce qu'on mélange des dérivés d'amides d'amino-acides de formule (I) suivant les revendications 1 à 5 avec des diluants et/ou des agents tensio-actifs.

**10.** Dérivés d'amino-acides de formule (II) ou leurs dérivés activés sur la fonction carboxy

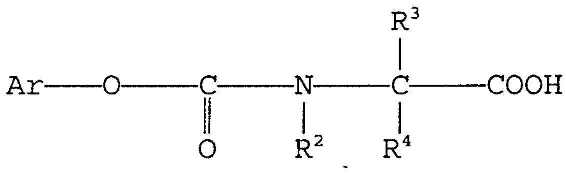

(II)

dans laquelle

Ar est un groupe aryle non substitué ou substitué, un groupe aralkyle non substitué ou substitué, un groupe hétéroaryle non substitué ou substitué ou un groupe hétéroarylalkyle non substitué ou substitué,

$R^2$ et $R^4$ sont identiques ou différents et représentent de l'hydrogène ou un groupe alkyle et

$R^3$ est un groupe cycloalkyle,

excepté les composés ester N-benzylique d'acide α-(carbonylamino)cyclohexane-acétique, ester 4-nitrophénylique d'acide α-[[(phénylméthoxy)carbonyl]amino]-(S)-cyclohexaneacétique, ester 4-nitrophénylique d'acide α-[[(phénylméthoxy)carbonyl]amino]-(5)-cyclohexane-acétique, acide α-[[(phénylméthoxy)carbonyl]amino]-(R)-cyclohexane-acétique, acide α-[[(phénylméthoxy)carbonyl]amino]-(S)-cyclohexaneacétique et ester N-benzylique d' acide α-(carboxyamino)-(L)-cyclohexane-acétique.

**11.** Procédé de production de dérivés d'amino-acides de formule (II) ou de leurs esters activés sur la fonction carboxy suivant la revendication 10, caractérisé en ce qu'on fait réagir des dérivés d'amino-acides de formule (IV)

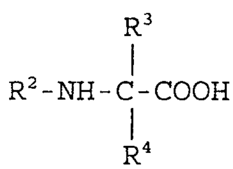

(IV)

dans laquelle

$R^2$, $R^3$ et $R^4$ ont la définition indiquée dans la revendication 1

avec des chlorures d'acides de formule (V)

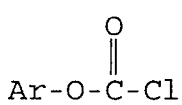

(V)

dans laquelle

Ar a la définition indiquée dans la revendication 10,

le cas échéant en présence d'un accepteur d'acide et en la présence éventuelle d'un diluant, à des températures de -10 °C à +10 °C.